(19) Europäisches Patentamt
European Patent Office
Office européen des brevets

(11) Veröffentlichungsnummer: **0 320 733 B1**

(12) **EUROPÄISCHE PATENTSCHRIFT**

(45) Veröffentlichungstag der Patentschrift: **03.03.93**

(51) Int. Cl.$^5$: **C07D 413/12**, A01N 43/80

(21) Anmeldenummer: **88120236.0**

(22) Anmeldetag: **03.12.88**

# (54) 2-tert.-Butyl-5-isoxazolylmethylthio-3(2H)-pyridazin-3-on-derivate.

(30) Priorität: **12.12.87 DE 3742266**

(43) Veröffentlichungstag der Anmeldung:
**21.06.89 Patentblatt 89/25**

(45) Bekanntmachung des Hinweises auf die Patenterteilung:
**03.03.93 Patentblatt 93/09**

(84) Benannte Vertragsstaaten:
**AT BE CH DE ES FR GB GR IT LI NL**

(56) Entgegenhaltungen:
**EP-A- 199 281**
**EP-A- 0 134 439**

(73) Patentinhaber: **BASF Aktiengesellschaft**
**Carl-Bosch-Strasse 38**
**W-6700 Ludwigshafen(DE)**

(72) Erfinder: **Leyendecker, Joachim, Dr.**
**Scharnhorststrasse 12**
**W-6800 Mannheim 51(DE)**
Erfinder: **Buerstinghaus, Rainer, Dr.**
**Robert-Koch-Strasse 2**
**W-4404 Telgte(DE)**
Erfinder: **Theobald, Hans, Dr.**
**Oueichstrasse 6**
**W-6703 Limburgerhof(DE)**
Erfinder: **Kuenast, Christoph, Dr.**
**Salierstrasse 2**
**W-6701 Otterstadt(DE)**
Erfinder: **Hofmeister, Peter, Dr.**
**Bernard-Humbolt-Strasse 12**
**W-6730 Neustadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.5x/3.0.1)

## Beschreibung

Die vorliegende Erfindung betrifft neue 2-tert.-Butyl-5-isoxazolylmethylthio-3(2H)-pyridazin-3-on-derivate der allgemeinen Formel I

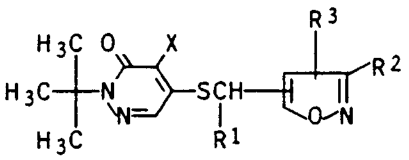

(I),

in der die Substituenten die folgende Bedeutung haben:

$R^1$ Wasserstoff oder $C_1$-$C_4$-Alkyl,

$R^2$ $C_3$-$C_{10}$-Cycloalkyl, Phenyl, Naphthyl, $C_7$-$C_{12}$-Phenylalkyl, ein- bis dreifach durch Halogen, $C_1$-$C_8$-Alkyl, $C_1$-$C_8$-Alkoxy, $C_1$-$C_4$-Halogenalkyl, $C_1$-$C_4$-Halogenalkoxy, Phenyl, Phenoxy, Cyano und/oder ein- bis zweifach durch Nitro substituiertes Phenyl oder Naphthyl oder im Phenylteil substituiertes $C_7$-$C_{12}$-Phenylalkyl,

$R^3$ Wasserstoff, Halogen, $C_1$-$C_8$-Alkyl oder $C_2$-$C_8$-Alkenyl und

X Chlor oder Brom.

Außerdem betrifft die Erfindung die Herstellung der Verbindungen I, Schädlingsbekämpfungsmittel, die die Verbindungen I als Wirkstoffe enthalten, sowie ein Verfahren zur Bekämpfung von Schädlingen.

Aus EP-A-199 281 und EP-A-134 439 sind zahlreiche hetarylisch oder durch Phenyl substituierte 3(2H)-Pyridazinonderivate bekannt, deren insektizide und akarizide Wirkung jedoch zu wünschen übrig läßt. Die dort beschriebenen Verbindungen enthalten als Hetarylrest keinen Isoxazolrest.

Der Erfindung lag daher die Aufgabe zugrunde, neue heterocyclisch substituierte 3(2H)-Pyridazinonderivate mit verbesserter Wirkung bereitzustellen.

Demgemäß wurden die eingangs definierten neuen 2-tert.-Butyl-5-isoxazolylmethylthio-3(2H)-pyridazin-3-on-derivate der allgemeinen Formel I sowie Verfahren zu deren Herstellung gefunden. Ferner wurde gefunden, daß sich die Verbindungen I bei sehr guter Pflanzenverträglichkeit zur Bekämpfung von Schädlingen eignen.

Die Substituenten in Formel I haben im einzelnen folgende Bedeutungen:

$R^1$
- Wasserstoff
- unverzweigtes oder verzweigtes $C_1$-$C_4$-Alkyl, bevorzugt $C_1$-$C_2$-Alkyl, besonders bevorzugt Methyl,

$R^2$
- $C_3$-$C_{10}$-Cycloalkyl, bevorzugt Cyclopropyl, Cyclobutyl, Cyclopentyl, Cyclohexyl, Cyclooctyl und Cyclodecyl,
- Phenyl und Naphthyl, besonders bevorzugt Phenyl,
- $C_7$-$C_{12}$-Phenylalkyl, wie Benzyl und Phenethyl,
- ein- bis dreifach durch Halogen substituiertes Phenyl und Naphthyl,
- bevorzugt durch Fluor, Chlor oder Brom ein- bis dreifach substituiertes Phenyl, wie 2-Fluorphenyl, 2-Chlorphenyl, 2-Bromphenyl, 3-Fluorphenyl, 3-Chlorphenyl, 3-Bromphenyl, 4-Fluorphenyl, 4-Chlorphenyl, 4-Bromphenyl, 2,3-Difluorphenyl, 2,3-Dichlorphenyl, 2,4-Difluorphenyl, 2,4-Dichlorphenyl, 2-Chlor-4-fluorphenyl, 4-Chlor-2-fluorphenyl, 2,5-Difluorphenyl, 2,5-Dichlorphenyl, 3,4-Difluorphenyl, 3,4-Dichlorphenyl, 3,5-Difluorphenyl, 3,5-Dichlorphenyl, 3-Chlor-5-fluorphenyl, 5-Chlor-3-fluorphenyl, 2,6-Difluorphenyl, 2,6-Dichlorphenyl, 2-Chlor-6-fluorphenyl, 6-Chlor-2-fluorphenyl, 2,3,4-Trifluorphenyl, 2,3,4-Trichlorphenyl, 2,4,6-Trifluorphenyl und 2,4,6-Trichlorphenyl,
- ein- bis dreifach durch $C_1$-$C_8$-Alkyl substituiertes Phenyl und Naphthyl, bevorzugt ein- bis dreifach durch $C_1$-$C_4$-Alkyl substituiertes Phenyl, wie 2-Methylphenyl, 3-Methylphenyl, 4-Methylphenyl, 2-Ethylphenyl, 3-Ethylphenyl, 4-Ethylphenyl, 2-n-Propylphenyl, 3-n-Propylphenyl, 4-n-Propylphenyl, 3-iso-Propylphenyl, 4-iso-Propylphenyl, 2-n-Butylphenyl, 3-n-Butylphenyl, 4-n-Butylphenyl, 3-iso-Butylphenyl, 4-iso-Butylphenyl, 3-sec.-Butylphenyl, 4-sec.-Butylphenyl, 3-tert.-Butylphenyl, 4-tert.-Butylphenyl, 2,3-Dimethylphenyl, 2,4-Dimethylphenyl, 2,5-Dimethylphenyl, 3,4-Dimethylphenyl, 3,5-Dimethylphenyl, 2,6-Dimethylphenyl, 3,6-Dimethylphenyl, 2,4-Diethylphenyl, 2,4-Di-iso-propylphenyl, 3,5-Di-tert.-butylphenyl, 2,6-Di-tert.-butylphenyl, 2,4,6-Trimethylphenyl, 2,3,4-Trimethylphenyl, 2,3,5-Trimethylphenyl, 3,4,5-Trimethylphenyl,
- ein- bis dreifach durch $C_1$-$C_8$-Alkoxy substituiertes Phenyl und Naphthyl, bevorzugt durch $C_1$-$C_8$-Alkoxy substituiertes Phenyl, wie 2-Methoxyphenyl, 3-Methoxyphenyl, 4-Methoxyphenyl, 2-Ethoxyphenyl, 3-Ethoxyphenyl, 4-Ethoxyphenyl, 2-n-Propoxyphenyl, 3-n-Propoxyphenyl, 4-n-Propoxyphenyl, 3-iso-Propoxyphenyl, 4-iso-Propoxyphenyl, 2-n-Butoxyphenyl, 3-n-Butoxyphenyl, 4-n-Butoxyphenyl, 2-sec-Butoxyphenyl, 4-sec-Butoxyphenyl, 2-tert.-Butoxyphenyl, 4-tert.-Butoxyphenyl, 2-n-Hexoxyphenyl, 3-n-Hexoxyphenyl, 4-n-Hexoxyphenyl, 2-n-Octoxyphenyl, 3-n-Octoxyphenyl, 4-n-Octoxyphenyl, 2,3-Dimethoxyphenyl, 2,4-Dimethoxyphenyl, 3,4-Dimethoxyphenyl, 2,5-Dimethoxyphenyl, 3,5-Dimethoxyphenyl, 2,6-Dimethoxyphenyl, 3,4-Diethoxyphenyl, 2,3,4-Trimethoxyphenyl, 2,3,5-Trimethoxyphenyl, 2,4,5-Trimethoxyphenyl, 2,4,6-Trimethoxyphenyl, 3,4,5-Trimethoxyphenyl,

- ein- bis dreifach durch $C_1$-$C_4$-Halogenalkyl substitutiertes Phenyl und Naphthyl, bevorzugt durch Fluor und/oder Chlor ein- bis fünffach substituiertes $C_1$-$C_2$-Alkyl monosubstituiert am Phenyl, wie 2-Fluormethylphenyl, 3-Fluormethylphenyl, 4-Fluormethylphenyl, 2-Difluormethylphenyl, 3-Difluormethylphenyl, 4-Difluormethylphenyl, 2-Trifluormethylphenyl, 3-Trifluormethylphenyl, 4-Trifluormethylphenyl, 2-Chlormethylphenyl, 3-Chlormethylphenyl, 4-Chlormethylphenyl, 2-Dichlormethylphenyl, 3-Dichlormethylphenyl, 4-Dichlormethylphenyl, 2-Trichlormethylphenyl, 3-Trichlormethylphenyl, 4-Trichlormethylphenyl, 2-Chlorfluormethylphenyl, 3-Chlorfluormethylphenyl, 4-Chlorfluormethylphenyl, 2-Dichlorfluormethylphenyl, 3-Dichlorfluormethylphenyl, 4-Dichlorfluormethylphenyl, 2-Chlordifluormethylphenyl, 3-Chlordifluormethylphenyl, 4-Chlordifluormethylphenyl, 1,1-Difluorethylphenyl, 1,1-Dichlorethylphenyl, ortho-, meta- oder para-1,1,2-Trifluorethylphenyl, ortho-, meta- oder para-1,1,2-Trichlorethylphenyl, ortho-, meta- oder para-1,1,2,2-Tetrafluorethylphenyl, ortho-, meta- oder para-1,1,2,2-Tetrachlorethylphenyl, ortho-, meta- oder para-1,2,2,2-Tetrafluorethylphenyl, ortho-, meta- oder para-1,2,2,2-Tetrachlorethylphenyl, ortho-, meta- oder para-Pentafluorethylphenyl und ortho-, meta- oder para-Pentachlorethylphenyl,

- ein- bis dreifach durch $C_1$-$C_4$-Halogenalkoxy substituiertes Phenyl und Naphthyl, bevorzugt durch Fluor und/oder Chlor ein- bis fünffach substituiertes $C_1$-$C_2$-Alkoxy monosubstituiert am Phenyl, wie 2-Fluormethoxyphenyl, 3-Fluormethoxyphenyl, 4-Fluormethoxyphenyl, 2-Difluormethoxyphenyl, 3-Difluormethoxyphenyl, 4-Difluormethoxyphenyl, 2-Trifluormethoxyphenyl, 3-Trifluormethoxyphenyl, 4-Trifluormethoxyphenyl, 2-Chlormethoxyphenyl, 3-Chlormethoxyphenyl, 4-Chlormethoxyphenyl, 2-Dichlormethoxyphenyl, 3-Dichlormethoxyphenyl, 4-Dichlormethoxyphenyl, 2-Trichlormethoxyphenyl, 3-Trichlormethoxyphenyl, 4-Trichlormethoxyphenyl, 2-Chlorfluormethoxyphenyl, 3-Chlorfluormethoxyphenyl, 4-Chlorfluormethoxyphenyl, 2-Dichlorfluormethoxyphenyl, 3-Dichlorfluormethoxyphenyl, 4-Dichlorfluormethoxyphenyl, 2-Chlordifluormethoxyphenyl, 3-Chlordifluormethoxyphenyl, 4-Chlordifluormethoxyphenyl, 1,1-Difluorethoxyphenyl, 1,1-Dichlorethoxyphenyl, ortho-, meta- oder para-1,1,2-Trifluorethoxyphenyl, ortho-, meta- oder para-1,1,2-Trichlorethoxyphenyl, ortho-, meta- oder para-1,1,2,2-Tetrafluorethoxyphenyl, ortho-, meta- oder para-1,1,2,2-Tetrachlorethoxyphenyl, ortho-, meta- oder para-1,2,2,2-Tetrafluorethoxyphenyl, ortho-, meta- oder para-1,2,2,2-Tetrachlorethoxyphenyl, ortho-, meta- oder para-Pentafluorethoxyphenyl und ortho-, meta- oder para-Pentachlorethoxyphenyl,

- ein- bis dreifach durch Phenyl substituiertes Phenyl und Naphthyl, bevorzugt durch Phenyl monosubstituiertes Phenyl, wie 2-Phenylphenyl, 3-Phenylphenyl und 4-Phenylphenyl,

- ein- bis dreifach durch Phenoxy substituiertes Phenyl und Naphthyl, bevorzugt durch Phenoxy monosubstituiertes Phenyl, wie 2-Phenoxyphenyl, 3-Phenoxyphenyl und 4-Phenoxyphenyl,

- ein- bis dreifach durch Cyano substituiertes Phenyl und Naphthyl, bevorzugt durch Cyano monosubstituiertes Phenyl, wie 2-Cyanophenyl, 3-Cyanophenyl und 4-Cyanophenyl,

- ein- bis zweifach durch Nitro substituiertes Phenyl und Naphthyl, bevorzugt durch Nitro mono- oder disubstituiertes Phenyl, wie 2-Nitrophenyl, 3-Nitrophenyl, 4-Nitrophenyl, 2,3-Dinitrophenyl, 2,4-Dinitrophenyl, 3,4-Dinitrophenyl, 2,5-Dinitrophenyl, 3,5-Dinitrophenyl und 2,6-Dinitrophenyl,

- zwei- bis dreifach durch Halogen und Nitro substituiertes Phenyl und Naphthyl, bevorzugt zwei- bis dreifach durch Fluor und/oder Chlor und Nitro substituiertes Phenyl, wie 2-Fluor-3-nitrophenyl, 2-Fluor-4-nitrophenyl, 2-Fluor-5-nitrophenyl, 2-Fluor-6-nitrophenyl, 2-Chlor-3-nitrophenyl, 2-Chlor-4-nitrophenyl, 2-Chlor-5-nitrophenyl, 2-Chlor-6-nitrophenyl, 3-Chlor-2-nitrophenyl, 4-Chlor-2-nitrophenyl, 4-Chlor-3-nitrophenyl, 5-Chlor-2-nitrophenyl, 2,4-Dichlor-5-nitrophenyl, 2,6-Dichlor-5-nitrophenyl, 2,6-Dichlor-3-nitrophenyl, 2,4-Dichlor-3-nitrophenyl,

- zwei- bis dreifach durch $C_1$-$C_4$-Alkyl und Nitro substituiertes Phenyl und Naphthyl, bevorzugt zwei- bis dreifach durch $C_1$-$C_2$-Alkyl und Nitro substituiertes Phenyl, wie 2-Methyl-3-nitrophenyl, 4-Methyl-3-nitrophenyl, 2,6-Dimethyl-3-nitrophenyl,

- zwei- bis dreifach durch Halogen und $C_1$-$C_4$-Alkoxy substituiertes Phenyl und Naphthyl, bevorzugt zwei- bis dreifach durch Brom und $C_1$-$C_2$-Alkoxy substituiertes Phenyl, wie 3-Brom-2-methoxyphenyl, 4-Brom-2-methoxyphenyl, 5-Brom-2-methoxyphenyl, 2-Brom-4-methoxyphenyl, 3-Brom-4-methoxyphenyl,

- im Phenylteil ein- bis dreifach durch Halogen substituiertes $C_7$-$C_{12}$-Phenylalkyl, bevorzugt im Phenylteil durch Fluor oder Chlor monosubstituiertes $C_7$-$C_{10}$-Phenyl-alkyl, wie 4-Fluorbenzyl und 4-Chlorbenzyl,
- im Phenylteil ein- bis dreifach durch $C_1$-$C_8$-Alkyl substituiertes $C_7$-$C_{12}$-Phenylalkyl, bevorzugt im Phenylteil durch $C_1$-$C_4$-Alkyl monosubstituiertes $C_7$-$C_{10}$-Phenyl-alkyl, besonders bevorzugt im Phenylteil durch $C_1$-$C_2$-Alkyl monosubstituierts $C_7$-$C_{10}$-Phenylalkyl, wie 4-Methylphenyl, 4-Ethylbenzyl, 4-Methylphenethyl und 2(4-tert.-Butylphenyl)-1-methylethyl,
- im Phenylteil ein- bis dreifach durch $C_1$-$C_8$-Alkoxy substituiertes $C_7$-$C_{12}$-Phenylalkyl, bevorzugt im Phenylteil durch $C_1$-$C_4$-Alkoxy monosubstituiertes $C_7$-$C_{10}$-Phenylalkyl, besonders bevorzugt im Phenylteil durch $C_1$-$C_2$-Alkoxy monosubstituiertes $C_7$-$C_{10}$-Phenylalkyl, wie 4-Methoxybenzyl, 4-Ethoxybenzyl und 4-Methoxyphenethyl,
- im Phenylteil ein- bis dreifach durch $C_1$-$C_4$-Halogenalkyl, substituiertes $C_7$-$C_{12}$-Phenylalkyl, bevorzugt im Phenylteil $C_1$-$C_4$-Fluor- oder Chloralkyl monosubstituiertes $C_7$-$C_{10}$-Phenylalkyl, besonders bevorzugt im Phenylteil $C_1$-$C_2$-Fluor- oder Chloralkyl monosubstituiertes $C_7$-$C_{10}$-Phenylalkyl wie 4-Trifluormethylbenzyl und 4-Trichlormethylbenzyl,
- im Phenylteil ein- bis dreifach durch $C_1$-$C_4$-Halogenalkoxy substituiertes $C_7$-$C_{12}$-Phenylalkyl, bevorzugt im Phenylteil durch $C_1$-$C_2$-Halogenalkyl monosubstituiertes $C_7$-$C_{10}$-Phenyl-alkyl, besonders bevorzugt durch Trifluormethyl und Trichlormethyl monosubstituiertes $C_7$-$C_{10}$-Phenylalkyl wie 4-Trifluormethoxybenzyl und 4-Trichlormethoxybenzyl,
- im Phenylteil ein- bis dreifach durch Cyano substituiertes $C_7$-$C_{12}$-Phenylalkyl, bevorzugt im Phenylteil durch Cyano monosubstituiertes $C_7$-$C_{10}$-Phenyl-alkyl, wie 4-Cyanobenzyl und 4-Cyanophenethyl, im
- Phenylteil ein- bis zweifach durch Nitro substituiertes $C_7$-$C_{12}$-Phenylalkyl, bevorzugt durch Nitro monosubstituiertes $C_7$-$C_{10}$-Phenylalkyl wie 3-Nitrobenzyl,

$R^3$
- Wasserstoff, bevorzugt Wasserstoff in 4-Position,
- Halogen, bevorzugt Fluor, Chlor und Brom, besonders bevorzugt Brom in 5-Position,
- unverzweigtes oder verzweigtes $C_1$-$C_8$-Alkyl, bevorzugt unverzweigtes oder verzweigtes $C_1$-$C_4$-Alkyl, besonders bevorzugt Methyl in 5-Position,
- unverzweigtes oder verzweigtes $C_2$-$C_8$-Alkenyl, bevorzugt unverzweigtes oder verzweigtes $C_2$-$C_4$-Alkenyl, besonders bevorzugt Vinyl, Methylvinyl und Dimethylvinyl,

X
- Chlor oder Brom, bevorzugt Chlor.

Die Verbindungen I sind nach folgender Methode erhältlich:

Die Umsetzung erfolgt zwischen 2-tert.-Butyl-5-mercapto-3(2H)-pyridazinonen der Formel II und einem Isoxazol der allgemeinen Formel III in Gegenwart einer Base im Temperaturbereich von (-20) bis 250 °C, vorzugsweise von 20 bis 120 °C nach folgender Reaktionsgleichung:

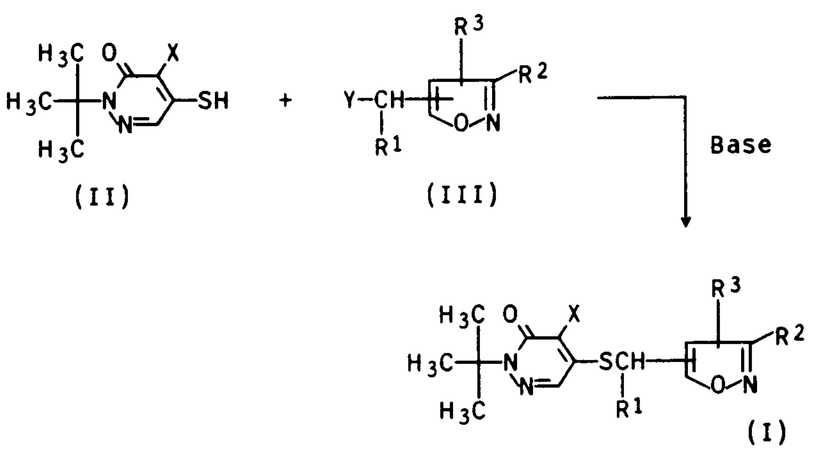


(II) (III) (I)

2-tert.-Butyl-5-mercapto-3(2H)-pyridazinone der Formel II sind aus EP-A-134 439 bekannt und können nach den dort beschriebenen Methoden hergestellt werden.

Die Isoxazole der allgemeinen Formel III sind zum Teil aus DE-A-25 49 962 und DE-A-27 54 832 bekannt oder können nach den dort beschriebenen Methoden erhalten werden.

Der Rest Y bedeutet eine Abgangsgruppe wie beispielsweise einen Sulfonsäurerest oder ein Halogen. Unter den Sulfonsäureestern sind Methansulfonyl, Trifluormethansulfonyl, Benzolsulfonyl und p-Toluolsulfonyl, unter den Halogenen sind Chlor und Brom bevorzugt; besonders bevorzugt wird Chlor.

Zur Herstellung der erfindungsgemäßen Verbindungen I nach den vorstehend beschriebenen Methoden setzt man die Ausgangsstoffe üblicherweise in stöchiometrischem Verhältnis ein. Ein Überschuß der einen oder anderen Komponente kann gegebenenfalls von Vorteil sein.

Die Umsetzungen verlaufen gewöhnlich oberhalb von -20 °C mit ausreichenden Geschwindigkeiten. 120 °C müssen im allgemeinen nicht überschritten werden. Da sie in einigen Fällen unter Wärmeentwicklung verlaufen, kann es von Vorteil sein, Kühlmöglichkeiten vorzusehen.

Man setzt normalerweise mindestens äquivalente Mengen einer Base zu II und/oder III zu, kann aber diese auch im Überschuß oder gegebenenfalls auch als Lösungsmittel verwenden. Als Basen eignen sich beispielsweise Hydroxide von Alkali- und Erdalkalimetallen, wie Natriumhydroxid, Kaliumhydroxid und Calciumhydroxid, Alkoholate von Alkali- und Erdalkalimetallen, wie Natriummethylat, Natriumethylat, Calciummethanolat oder Kalium-tert.-butylat; Alkali- oder Erdalkalimetallhydride, wie Natriumhydrid, Kaliumhydrid oder Calciumhydrid; Alkali- oder Erdalkalicarbonate, wie Natriumcarbonat, Kaliumcarbonat oder Calciumcarbonat; aliphatische Amine, wie Dimethylamin, Triethylamin oder Diisopropylamin; heterocyclische Amine, wie Piperidin, Piperazin oder Pyrrolidin; aromatische Amine, wie Pyridin oder Pyrrol.

Die Umsetzung wird zweckmäßigerweise in einem Lösungsmittel oder Verdünnungsmittel durchgeführt. Hierzu eignen sich bespielsweise aliphatische Kohlenwasserstoffe, wie n-Pentan, n-Hexan, das Hexan-Isomerengemisch und Petrolether; halogenierte Kohlenwasserstoffe wie Chlorbenzol, Methylenchlorid, Ethylenchlorid, Chloroform, Tetrachlorethylen; aromatische Kohlenwasserstoffe, wie Benzol, Toluol, die Xylole und deren Isomerengemische, Benzin; Alkohole, wie Methanol, Ethanol, n-Propanol und Isopropanol; Ether, wie Diethyl-, Di-n-butylether, Methyl-tert.-butylether, Tetrahydrofuran und Dioxan; Ketone, wie Aceton, Methylethylketon und Methylisopropylketon: Nitrile, wie Acetonitril und Propionitril; aprotische dipolare Lösungsmittel wie Dimethylformamid, Dimethylsulfoxid oder Pyridin. Auch Gemische dieser Stoffe können als Lösungs- und Verdünnungsmittel verwendet werden.

Zweckmäßigerweise wird das 2-tert.-Butyl-5-mercapto-3(2H)-pyridazinonder Formel II in einem Verdünnungs- oder Lösungsmittel vorgelegt, unter anschließender Zugabe des Isoxazols III. Zur Isolierung der neuen Verbindungen I wird nach üblichen Methoden vorgegangen. Die anfallenden Produkte können durch Umkristallisieren, Extrahieren oder Chromatographieren gereinigt werden.

Die 2-tert.-Butyl-5-isoxazolylmethylthio-3(2H)-pyridazin-3-onderivate der allgemeinen Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Zur Klasse der Nematoden zählen beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Hetrodera glycinae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Paratylenchus curvitatus, Partylenchus goodeyi.

Die Wirkstoffe können als solche, in Form ihrer Formulierungen oder den daraus bereiteten Anwendungsmöglichkeiten, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkali, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Aklylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Beispiele für Formulierungen sind:

I. 5 Gew.-Teile der Verbindung Nr. 47 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 25 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 57 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 47 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 108 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Die Wirkstoffkonzentrationen in den anwendungsfertigen Zubereitungen können in größeren Bereichen variiert werden. Im allgemeinen liegen sie zwischen 0,0001 und 10 %, vorzugsweise zwischen 0,01 und 1 %. Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,001 bis 10, insbesondere 0,05 bis 2, vorzugsweise 0,05 bis 0,5 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Herstellungsbeispiel

2-tert.-Butyl-4-chlor-5-[(3-isopropyl-isoxazol-5-yl)-methylthio]-3(2H)-pyridazin-3-on

Zu 8,3 g (0,038 Mol) 2-tert.-Butyl-4-chlor-5-mercapto-3-(2H)-pyrdazinon und 5,2 g (0,038 Mol) Kaliumcarbonat in 50 ml Dimethylformamid gibt man bei Raumtemperatur (20 °C) 6,1 g (0,038 Mol) 5-Chlormethyl-3-isopropyl-isoxazol. Anschließend rührt man 2 Stunden bei 80 °C und über Nacht bei Raumtemperatur nach. Zur Aufarbeitung gießt man in 200 ml Wasser und extrahiert mit Essigsäureethylester. Die organische Phase wird 2 mal mit Wasser gewaschen, über Magnesiumsulfat getrocknet, eingeengt und der Rückstand aus n-Hexan/Essigester = 4/1 umkristallisiert. Man erhält 8,5 g (66 %) 2-tert.-Butyl-4-chlor-5-[(3-isopropyl-isoxazol-5-yl)-methylthio]-3(2H)-pyridazin-3-on als farblose Kristalle vom Schmp. 94-96 °C.

Die in den nachstehenden Tabellen 1 und 2 aufgeführten Verbindungen I können nach dem erfindungsgemäßen Verfahren aus entsprechenden Vorprodukten ohne weiteres erhalten werden.

Tabelle 1

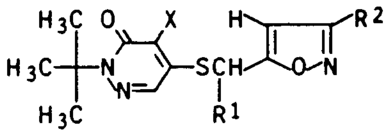

(Ia)

| Verb. Nr. | X | $R^1$ | $R^2$ | Fp. [°C] |
|---|---|---|---|---|

Tabelle 1: Fortsetzung

| Verb. Nr. | X | $R^1$ | $R^2$ | Fp. [°C] |
|---|---|---|---|---|
| 1 | Cl | H | Cyclopropyl | 126-127 |
| 2 | Br | H | Cyclopropyl | |
| 3 | Cl | H | Cyclopentyl | |
| 4 | Cl | H | Cyclohexyl | 88- 92 |
| 5 | Cl | $CH_3$ | Cyclohexyl | |
| 6 | Br | H | Cyclohexyl | |
| 7 | Cl | H | Benzyl | |

Tabelle 1: Fortsetzung

| Verb. Nr. | X | $R^1$ | $R^2$ | Fp. [°C] |
|---|---|---|---|---|
| 8 | Cl | H | Phenethyl | |
| 9 | Cl | $CH_3$ | 2-(4-tert.-butylphenyl)--1-methylethyl | Harz |
| 10 | Cl | H | Phenyl | 108-111 |
| 11 | Cl | $CH_3$ | Phenyl | 50- 52 |
| 12 | Br | H | Phenyl | |
| 13 | Br | $CH_3$ | Phenyl | |
| 14 | Cl | H | (2-Methyl)-phenyl | |
| 15 | Cl | H | (3-Methyl)-phenyl | |
| 16 | Cl | H | (4-Methyl)-phenyl | 150-154 |
| 17 | Cl | H | (2-Ethyl)-phenyl | |
| 18 | Cl | H | (4-Ethyl)-phenyl | |
| 19 | Cl | H | (4-iso-Propyl)-phenyl | |
| 20 | Cl | H | (4-tert. Butyl)-phenyl | 127-128 |
| 21 | Cl | H | (2,4-Dimethyl)-phenyl | |
| 22 | Cl | H | (3,5-Di-tert.-butyl)-phenyl | |
| 23 | Cl | H | (2,4,6-Trimethyl)-phenyl | |
| 24 | Cl | H | (4-Phenyl)-phenyl | 136-140 |
| 25 | Cl | $CH_3$ | (4-Phenyl)-phenyl | |
| 26 | Cl | H | (2-Methoxy)-phenyl | |
| 27 | Cl | $CH_3$ | (2-Methoxy)-phenyl | |
| 28 | Cl | H | (3-Methoxy)-phenyl | |
| 29 | Cl | H | (4-Methoxy)-phenyl | 148-150 |
| 30 | Cl | $CH_3$ | (4-Methoxy)-phenyl | |
| 31 | Cl | H | (2-Ethoxy)-phenyl | |
| 32 | Cl | H | (3-Ethoxy)-phenyl | |
| 33 | Cl | H | (4-Ethoxy)-phenyl | |
| 34 | Cl | $CH_3$ | (4-Ethoxy)-phenyl | |
| 35 | Cl | H | (4-tert.-Butoxy)-phenyl | |
| 36 | Cl | H | (2-n-Butoxy)-phenyl | 87- 88 |
| 37 | Cl | H | (4-n-Hexoxy)-phenyl | 70- 78 |
| 38 | Cl | H | (2-n-Octoxy)-phenyl | 70- 71 |
| 39 | Cl | $CH_3$ | (2-n-Octoxy)-phenyl | |
| 40 | Cl | H | (2,4-Dimethoxy)-phenyl | |
| 41 | Cl | H | (3,5-Dimethoxy)-phenyl | |
| 42 | Cl | H | (2,5-Dimethoxy)-phenyl | |
| 43 | Cl | H | (2,3-Dimethoxy)-phenyl | |

Tabelle 1: Fortsetzung

| Verb. Nr. | X | $R^1$ | $R^2$ | Fp. [°C] |
|---|---|---|---|---|
| 44 | Cl | H | (2,4,6-Trimethoxy)-phenyl | |
| 45 | Cl | H | (2,3,4-Trimethoxy)-phenyl | |
| 46 | Cl | H | (2,4,5-Trimethoxy)-phenyl | |
| 47 | Cl | H | (4-Trifluormethyl)-phenyl | 157-160 |
| 48 | Cl | $CH_3$ | (4-Trifluormethyl)-phenyl | |
| 49 | Cl | H | (2-Trifluormethyl)-phenyl | Harz |
| 50 | Cl | H | (3-Trifluormethyl)-phenyl | |
| 51 | Cl | H | (Chlor-difluormethyl)-phenyl | |
| 52 | Cl | H | (4-Difluormethoxy)-phenyl | |
| 53 | Cl | H | 2-(1,1,2,2-Tetrafluorethoxy)-phenyl | Harz |
| 54 | Cl | H | 3-(1,1,2,2-Tetrafluorethoxy)-phenyl | |
| 55 | Cl | H | 4-(1,1,2,2-Tetrafluorethoxy)-phenyl | |
| 56 | Cl | H | (2-Fluor)-phenyl | Harz |
| 57 | Cl | $CH_3$ | (2-Fluor)-phenyl | |
| 58 | Cl | H | (3-Fluor)-phenyl | 128-134 |
| 59 | Br | H | (3-Fluor)-phenyl | |
| 60 | Cl | H | (4-Fluor)-phenyl | 165-167 |
| 61 | Cl | $CH_3$ | (4-Fluor)-phenyl | |
| 62 | Cl | H | (2-Chlor)-phenyl | Harz |
| 63 | Cl | $CH_3$ | (2-Chlor)-phenyl | |
| 64 | Cl | H | (3-Chlor)-phenyl | |
| 65 | Cl | H | (4-Chlor)-phenyl | 166-170 |
| 66 | Cl | H | (3-Brom)-phenyl | |
| 67 | Cl | H | (3-Brom)-phenyl | |
| 68 | Cl | H | (4-Brom)-phenyl | |
| 69 | Cl | H | (2,6-Difluor)-phenyl | Harz |
| 70 | Cl | $CH_3$ | (2,6-Difluor)-phenyl | |
| 71 | Cl | H | (2-Chlor-6-fluor)-phenyl | 125-128 |
| 72 | Cl | $CH_3$ | (2-Chlor-6-fluor)-phenyl | |
| 73 | Br | H | (2-Chlor-6-fluor)-phenyl | |
| 74 | Cl | H | (2,3-Dichlor)-phenyl | |
| 75 | Cl | H | (3,4-Dichlor)-phenyl | 133-135 |
| 76 | Cl | $CH_3$ | (3,4-Dichlor)-phenyl | |
| 77 | Cl | H | (2,4-Dichlor)-phenyl | |
| 78 | Cl | H | (3,5-Dichlor)-phenyl | |
| 79 | Cl | H | (2,3,4-Trichlor)-phenyl | |
| 80 | Cl | H | (2-Chlor-6-nitro)-phenyl | |

Tabelle 1: Fortsetzung

| Verb. Nr. | X | $R^1$ | $R^2$ | Fp. [°C] |
|---|---|---|---|---|
| 81 | Cl | H | (4-Chlor-3-nitro)-phenyl | |
| 82 | Cl | H | (2-Chlor-5-nitro)-phenyl | |
| 83 | Cl | H | (5-Chlor-2-nitro)-phenyl | |
| 84 | Cl | H | (2,4-Dichlor-5-nitro)-phenyl | |
| 85 | Cl | H | (2,6-Dichlor-3-nitro)-phenyl | |
| 86 | Cl | H | (3-Brom-4-methoxy)-phenyl | 147-149 |
| 87 | Cl | $CH_3$ | (3-Brom-4-methoxy)-phenyl | |
| 88 | Cl | H | (5-Brom-2-methoxy)-phenyl | |
| 89 | Cl | H | (2-Nitro)-phenyl | |
| 90 | Br | H | (2-Nitro)-phenyl | |
| 91 | Cl | H | (3-Nitro)-phenyl | 127-129 |
| 92 | Cl | $CH_3$ | (3-Nitro)-phenyl | |
| 93 | Br | H | (3-Nitro)-phenyl | |
| 94 | Cl | H | (4-Nitro)-phenyl | |
| 95 | Cl | H | (2,4-Dinitro)-phenyl | |
| 96 | Cl | H | (4-Methyl-3-nitro)-phenyl | |
| 97 | Cl | H | (4-Phenoxy)-phenyl | 117-119 |
| 98 | Cl | $CH_3$ | (4-Phenoxy)-phenyl | |
| 99 | Br | H | (4-Phenoxy)-phenyl | |
| 100 | Cl | H | (2-Cyano)-phenyl | |
| 101 | Cl | $CH_3$ | (2-Cyano)-phenyl | 164-169 |
| 102 | Cl | H | (4-Cyano)-phenyl | |
| 103 | Cl | $CH_3$ | (4-Cyano)-phenyl | |
| 104 | Br | H | (4-Cyano)-phenyl | |

Tabelle 2

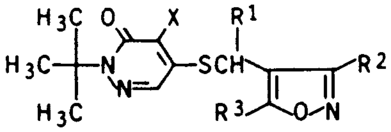

(Ib)

| Verb. Nr. | X | $R^1$ | $R^2$ | $R^3$ | Fp [°C] |
|---|---|---|---|---|---|
| 105 | Cl | H | Phenyl | H | |
| 106 | Cl | H | Phenyl | $CH_3$ | |
| 107 | Cl | H | Phenyl | Br | |
| 108 | Cl | H | (2-Fluor)-phenyl | $CH_3$ | |
| 109 | Cl | H | (4-Fluor)-phenyl | $CH_3$ | |
| 110 | Cl | H | (2-Chlor-6-fluor)-phenyl | $CH_3$ | |

Anwendungsbeispiele

In den folgenden Beispielen wurden die erfindungsgemäßen Verbindungen bzw. sie enthaltende Mittel hinsichtlich ihrer Wirkung auf Schädlinge mit der nachstehenden Verbindung des Standes der Technik bzw. diese enthaltenden Mitteln verglichen.

A: 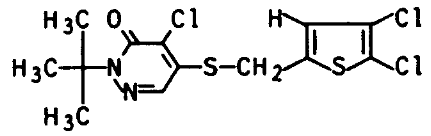 bekannt aus EP-A-199 281 als Verbindung Nr. 866

Die Konzentrationen, bei denen die untersuchten Verbindungen eine 100 %ige Mortalität bzw. Hemmung bewirken, sind die jeweiligen Minimalkonzentrationen. Bei jeder Konzentration wurden mindestens 2 Versuche angesetzt.

Beispiel A

Tetranychus telarius (Spinnmilbe), Kontaktwirkung, Spritzversuch

Getopfte Buschbohnen, die das erste Folgeblattpaar entwickelt haben und einen starken Besatz aller Stadien der Spinnmilbe Tetranychus telarius tragen, werden in der Spritzkabine mit der wäßrigen Wirkstoffaufbereitung tropfnaß gespritzt. Die Pflanzen kommen dazu auf einen Drehteller und werden von allen Seiten mit 50 ml Spritzbrühe besprüht. Der Sprühvorgang dauert ca. 22 Sekunden. Nach 8 Tagen werden die Pflanzen auf lebende Spinnmilben untersucht.

In diesem Versuch lag die mortale Dosis der Verbindung Nr. 4 bei 0,02 Gew.% und die der Verbindung Nr.20 bei 0,04 Gew.%. Die Verbindung Nr. 10 zeigte bei 0,004 Gew.% eine 80 %ige Mortalität und die Vergleichsverbindung A zeigte bei 0,1 Gew.% keine Wirkung (0 % Mortalität).

Beispiel B

Tetranychus telarius (Spinnmilbe), Versuch mit gezählten Weibchen

Getopfte Buschbohnen werden in der Spritzkabine auf dem Drehteller mit der wäßrigen Wirkstoffaufbereitung tropfnaß gespritzt. Nach dem Abtrocknen stanzt man mit einem Korkbohrer Blattstücke von 25 mm Durchmesser aus den Spreiten. Sie werden auf Zellstoffstücke gelegt, welche an den Seiten in Wasser hängen. Die Blattstanzstücke werden mit 10 adulten Weibchen belegt. Nach 10 Tagen wird die Mortalität bestimmt.

Bei diesem Versuch zeigte die Verbindung Nr. 10 bei 0,002 Gew.% eine 80 %ige Mortalität.

Beispiel C

Dysdercus intermedius (Baumwollwanze), Zuchtversuch

Petrischalen von 10 cm Durchmesser werden mit 1 ml der acetonischen Wirkstofflösung ausgekleidet. Nach Verdunsten des Lösungsmittels besetzt man die Schalen mit je 20 Larven des vorletzten Stadiums. Nach 24 Stunden überführt man die überlebenden Tiere in 1 l-Gläser, die 200 g sterilen Quarzsand (Korngröße 0 bis 3 mm) enthalten. Dieser Sand wurde vor Versuchsbeginn mit 25 ml der wäßrigen Wirkstoffaufbereitung angegossen. Als Futter bietet man gequollene Baumwollsamen, die wöchentlich gewechselt werden. Ebenfalls wöchentlich feuchtet man den Sand mit reinem Wasser an.

Die Versuchstemperatur beträgt 25 bis 27 ° C. Die Beobachtungszeit erstreckt sich bis zum Schlüpfen der Eier in den Kontrollen.

Bei diesem Versuch lag die mortale Dosis der Verbindung Nr. 10 bei 4 ppm. Die Vergleichsverbindung A zeigte bei 25 ppm keine Wirkung (0 % Mortalität).

Beispiel D

Dysdercus intermedius (Baumwollwanze), Ovizide Wirkung

Stecketiketten werden am oberen Rand mit Klebestreifenstücken (ca. 0,8 cm) beklebt. 24 Stunden vor Versuchsbeginn werden die Eier durch Eintauchen in das "Sammelgefäß" an dem Klebestreifenrand befestigt. Die Eier werden für ca. 5 Sekunden in die wäßrige Wirkstoffaufbereitung getaucht. Anschließend läßt man auf einem Filterpapier abtropfen. Dabei sollen die Eier nicht auf das Papier gelegt werden. Die zugeschnittenen Etiketten werden in Plastikpaletten gestellt, mit dem Klebestreifen nach oben. Eine halbe mit Wasser durchfeuchtete Watterolle wird in den Becher gelegt, um das Austrocknen zu vermeiden und die Palette mit einer Glasplatte abgedeckt. Nach dem Schlupf der Larven im Kontrollversuch (nach ca. 8 Tagen) wird bonitiert. Beurteilt wird die Schlupfhemmung in %.

Bei diesem Versuch zeigte die Verbindung Nr. 10 bei 0,01 Gew.% eine 80 %ige Schlupfhemmung, die Verbindung Nr. 71 bei 0,1 Gew.% eine 90 %ige Schlupfhemmung und die Vergleichsverbindung A zeigte bei 0,1 Gew.% keine Wirkung (0 % Schlupfhemmung).

Beispiel E

Plutella maculipennis (Kohlschabe), Wirkung auf die Eiablagen

Junge Kohlpflanzen mit 2 bis 3 Blattpaaren, die eine starke Eiablage der Kohlschabe tragen, werden mit der wäßrigen Wirkstoffemulsion tropfnaß gespritzt. Die Pflanzen werden darauf unter Gewächshausbedingungen gehalten und der Fraß der geschlüpften Raupen beobachtet.

Bei diesem Versuch zeigte die Verbindung Nr. 10 bei 0,002 Gew.% eine 85 %ige Fraßhemmung.

Beispiel F

Epilachna varivestis (Mexikanischer Bohnenkäfer), Kontaktwirkung

Petrischalen von 10 cm Durchmesser werden mit der acetonischen Wirkstoffaufbereitung ausgekleidet. Nach Verdunsten des Lösungsmittels belegt man die Schalen mit je 5 Larven (3 bis 4 mm). Nach 24 Stunden wird die Mortalitätsrate festgestellt.

Bei diesem Versuch zeigte die Verbindung Nr. 10 bei 0,06 mg eine 85 %ige Mortalität.

## Patentansprüche

1. 2-tert.-Butyl-5-isoxazolylmethylthio-3(2H)-pyridazin-3-on-derivate der allgemeinen Formel I

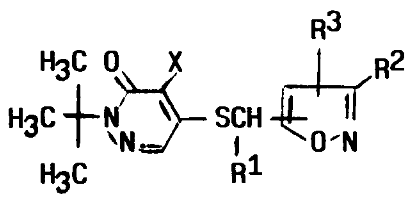

(I),

in der die Substituenten die folgende Bedeutung haben:

$R^1$ Wasserstoff oder $C_1$-$C_4$-Alkyl,

$R^2$ $C_3$-$C_{10}$-Cycloalkyl, Phenyl, Naphthyl, $C_7$-$C_{12}$-Phenylalkyl, ein- bis dreifach durch Halogen, $C_1$-$C_8$-Alkyl, $C_1$-$C_8$-Alkoxy, $C_1$-$C_4$-Halogenalkyl, $C_1$-$C_4$-Halogenalkoxy, Phenyl, Phenoxy, Cyano und/oder ein- bis zweifach durch Nitro substituiertes Phenyl oder Naphthyl oder im Phenylteil substituiertes $C_7$-$C_{12}$-Phenylalkyl,

$R^3$ Wasserstoff, Halogen, $C_1$-$C_8$-Alkyl oder $C_2$-$C_8$-Alkenyl und

X Chlor oder Brom.

2. Verfahren zur Herstellung der 2-tert.-Butyl-5-isoxazolylmethylthio-3(2H)-pyridazin-3-on-derivate der allgemeinen Formel I gemäß Anspruch 1, dadurch gekennzeichnet, daß man in an sich bekannter Weise

2-tert.-Butyl-5-mercapto-3(2H)-pyridazinone der allgemeinen Formel II

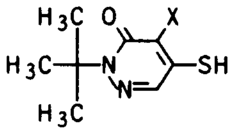

(II)

mit einem Isoxazol der allgemeinen Formel III

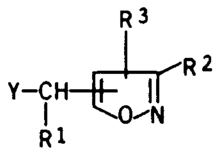

(III),

in der Y für eine Abgangsgruppe steht, in Gegenwart einer Base umsetzt.

3. Schädlingsbekämpfungsmittel, enthaltend ein 2-tert.-Butyl-5-isoxazolylmethylthio-3(2H)-pyridazin-3-on-derivat gemäß Anspruch 1 neben hierfür üblichen Trägerstoffen.

4. Schädlingsbekämpfungsmittel nach Anspruch 3, dadurch gekennzeichnet, daß es 0,1 bis 95 Gew.% eines 2-tert.-Butyl-5-isoxazolylmethylthio-3(2H)-pyridazin-3-on-derivats der Formel I enthält.

5. Verfahren zur Bekämpfung von Schädlingen, dadurch gekennzeichnet, daß man die Schädlinge und/oder die von Schädlingen freizuhaltenden Flächen und/oder Räume mit einer für Schädlinge wirksamen Menge eines 2-tert.-Butyl-5-isoxazolylmethylthio-3(2H)-pyridazin-3-on-derivats der Formel I gemäß Anspruch I behandelt.

## Claims

1. A 2-tert-butyl-5-isoxazolylmethylthio-3(2H)-pyridazin-3-one derivative of the formula I

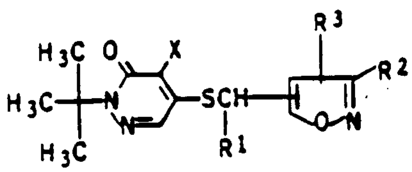

(I),

where $R^1$ is hydrogen or $C_1$-$C_4$-alkyl, $R^2$ is $C_3$-$C_{10}$-cycloalkyl, phenyl, napthyl or $C_7$-$C_{12}$-phenylalkyl, or is phenyl or naphthyl, each of which is mono-, di- or trisubstituted by halogen, $C_1$-$C_8$-alkyl, $C_1$-$C_8$-alkoxy, $C_1$-$C_4$-haloalkyl, $C_1$-$C_4$-haloalkoxy, phenyl, phenoxy or cyano, and/or mono- or disubstituted by nitro, or is $C_7$-$C_{12}$-phenylalkyl substituted in the phenyl moiety, $R^3$ is hydrogen, halogen, $C_1$-$C_8$-alkyl or $C_2$-$C_8$-alkenyl, and X is chlorine or bromine.

2. A process for the preparation of 2-tert-butyl-5-isoxazolylmethylthio-3(2H)-pyridazin-3-one derivatives of the general formula I as claimed in claim 1, wherein a 2-tert-butyl-5-mercapto-3(2H)-pyridazinone of the general formula II

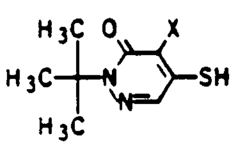

(II)

is reacted in conventional manner with an isoxazole of the general formula III

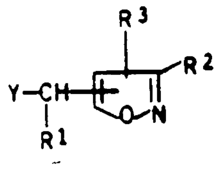

(III),

where Y is leaving group, in the presence of a base.

3. A pesticide containing a 2-tert-butyl-5-isoxazolylmethylthio-3(2H)-pyridazin-3-one derivative as claimed in claim 1 in addition to conventional carriers therefor.

4. A pesticide as claimed in claim 3, containing from 0.1 to 95% by weight of a 2-tert-butyl-5-isoxazolylmethylthio-3(2H)-pyridazin-3-one derivative of the formula I.

5. A method for controlling pests, wherein the pests or the areas or rooms to be kept free from pests are treated with a pesticidally effective amount of a 2-tert-butyl-5-isoxazolylmethylthio-3(2H)-pyridazin-3-one derivative of the formula I as claimed in claim 1.

**Revendications**

1. Dérivés de 2-tert-butyl-5-isoxazolylméthylthio-3(2H)-pyridazin-3-one de la formule générale I

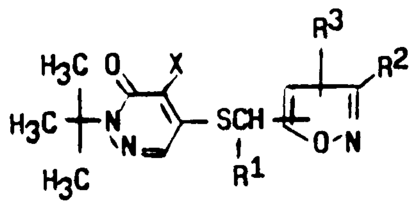

(I),

dans laquelle les substituants ont les significations suivantes :

$R^1$, hydrogène ou alkyle en C1-C4,

$R^2$, cycloalkyle en C3-C10, phényle, naphtyle, phénylalkyle en C7-C12, phényle ou naphtyle substitué une à trois fois par halogène, alkyle en C1-C8, alcoxy en C1-C8, halogénoalkyle en C1-C4, halogénoalcoxy en C1-C4, phényle, phénoxy, cyano et/ou une à deux fois par nitro, ou phénylalkyle en C7-C12 substitué dans la partie phényle.

$R^3$, hydrogène, halogène, alkyle en C1-C8 ou alcényle en C2-C8, et

X, chlore ou brome.

2. Procédé de préparation de dérivés de 2-tert-butyl-5-isoxazolylméthylthio-3(2H)-pyridazin-3-one de la formule générale I selon la revendication 1, caractérisé par le fait que l'on fait réagir, en présence d'une base, de manière connue en soi, une 2-tert-butyl-5-mercapto-3(2H)-pyridazinone de formule générale II

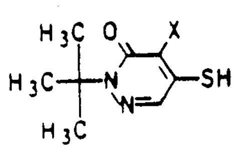

(II)

avec un isoxazole de la formule générale III

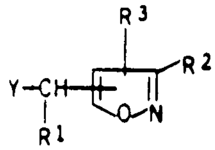

(III),

dans laquelle Y est mis pour un groupe éliminable.

3. Pesticide, contenant un dérivé de 2-tert-butyl-5-isoxazolylméthylthio-3(2H)-pyridazin-3-one selon la revendication 1, à côté de matières supports usuelles pour cela.

4. Pesticide selon la revendication 3, caractérisé par le fait qu'il contient 0,1 à 95 % en poids d'un dérivé de 2-tert-butyl-5-isoxazolylméthylthio-3(2H)-pyridazin-3-one de formule I.

5. Procédé de lutte contre les parasites, caractérisé par le fait que l'on traite les parasites et/ou les surfaces et/ou espaces à maintenir exempts de parasites avec une quantité, efficace pour les parasites, d'un dérivé de 2-tert-butyl-5-isoxazolylméthylthio-3(2H)-pyridazin-3-one de la formule I selon la revendication 1.